# EUROPÄISCHE PATENTSCHRIFT

(11) **EP 0 378 083 B1**
(45) Veröffentlichungstag und Bekanntmachung des Hinweises auf die Patenterteilung: **28.07.1993**
(21) Anmeldenummer: 90100059.6
(22) Anmeldetag: 03.01.1990
(51) Int. Cl.: C07C 11/24, C07C 2/78

(54) **Verfahren zur Herstellung von Acetylen durch unvollständige Verbrennung von Kohlenwasserstoffen**
Method for the production of acetylene by incomplete combustion of hydrocarbons
Procédé pour la fabrication d'acétylène par combustion incomplète d'hydrocarbures

(30) Priorität: 10.01.1989 DE 3900461
(43) Veröffentlichungstag der Anmeldung: 18.07.1990
(73) Patentinhaber: BASF Aktiengesellschaft, 67063 Ludwigshafen (DE)
(72) Erfinder: Reichert, Martin, Dr., D-6710 Frankenthal (DE); Wunsch, Gerd, Dr., D-6720 Speyer (DE); Paessler, Peter, Dr., D-6700 Ludwigshafen (DE); Arndt, Volker, Dr., D-6712 Bobenheim-Roxheim (DE); Kastenhuber, Werner, D-6704 Mutterstadt (DE)

(56) Entgegenhaltungen:
- DE-B- 1 643 625
- DE-C- 662 051
- US-A- 2 875 259

## Beschreibung

Die vorliegende Erfindung betrifft ein Verfahren zur Herstellung von Acetylen durch unvollständige Verbrennung von Kohlenwasserstoffen mit Sauerstoff.

Es ist bekannt, z.B. aus Ullmanns Encyklopädie der technischen Chemie, 4. Auflage, Band 7, Seiten 50 bis 52, Acetylen aus Methan bzw. Leichtbenzindampf oder Flüssiggas und Sauerstoff in der Weise herzustellen, daß der eingesetzte Kohlenwasserstoff und der Sauerstoff getrennt vorgewärmt werden, die vorgewärmten Gase gemischt werden und die Mischung einem Reaktionsraum zugeführt wird, in dem sich in einer Flammenreaktion durch unvollständige Verbrennung der Kohlenwasserstoffe acetylenhaltiges Spaltgas bildet, das anschließend nach Verlassen des Reaktionsraumes mittels einer Abschreckflüssigkeit wie Wasser oder aromatische Kohlenwasserstoffe rasch auf tiefe Temperaturen abgekühlt wird.

Die Vorwärmtemperatur vor der Reaktion der gasförmigen oder verdampften Kohlenwasserstoffe wird möglichst hoch gewählt, um einen möglichst geringen Verbrauch an den Kohlenwasserstoffen und Sauerstoff zu erzielen und um im Spaltgas eine möglichst hohe Acetylenkonzentration zu erreichen, was für die spätere Abtrennung des Acetylens wirtschaftlich von Vorteil ist. Die maximale Vorwärmtemperatur der eingesetzten Kohlenwasserstoffe ist abhängig von deren thermischen Stabilität und ist beim Methan mit ca. 650°C am höchsten, beträgt jedoch bei den höheren Kohlenwasserstoffen, z.B. beim Leichtbenzin, nur ca. 320°C.

Sollen unterschiedliche Kohlenwasserstoffe zum Einsatz kommen, sind neben den unterschiedlichen Aufheizbedingungen auch unterschiedliche Brenner erforderlich, die im Bereich der Mischeinrichtungen und der Brennkammer in ihren Dimensionen den unterschiedlichen Volumenströmen angepaßt sein müssen. Die Flexibilität im Einsatz unterschiedlicher Kohlenwasserstoffe in einer bestehenden Anlage macht daher die Vorhaltung unterschiedlicher Brennereinrichtungen oder einen kostspieligen, produktionsmindernden Umbau bestehender Brennereinrichtungen erforderlich.

Es bestand daher die Aufgabe, den Einsatz unterschiedlicher Kohlenwasserstoffe für die Herstellung von Acetylen durch unvollständige Oxidation der Kohlenwasserstoffe flexibler zu gestalten.

Es wurde nun ein vorteilhaftes Verfahren gefunden zur Herstellung von Acetylen durch unvollständige Verbrennung von Methan mit Sauerstoff, wobei das Methan und der Sauerstoff getrennt vorgewärmt und gemischt werden und die Mischung der vorgewärmten Gase in einer Flammenreaktion umgesetzt wird, welches dadurch gekennzeichnet ist, daß dem Methan höhere gas- und/oder dampfförmige Kohlenwasserstoffe in Mengen von 10 bis 70 Gew.% bezogen auf die Mischung aus Methan und den höheren gas- und/oder dampfförmigen Kohlenwasserstoffen, zugemischt werden und die Mischung aus Methan und den höheren Kohlenwasserstoffen auf mindestens 450°C vorgewärmt wird.

Nach dem neuen Verfahren können Methan und höhere gas- und/oder dampfförmige Kohlenwasserstoffe als Einsatzstoffe für die Acetylenherstellung durch unvollständige Verbrennung verwendet werden, ohne daß ein Umbau der Brennereinrichtungen erforderlich ist.

Überraschenderweise steigt bei der erfindungsgemäßen Verwendung einer Mischung von Methan und höheren gas- und/oder dampfförmigen Kohlenwasserstoffen als Einsatzstoff die Kapazität einer bestehenden Brennereinrichtung in bezug auf die Acetylenbildung stärker an als wie sie aufgrund der Kapazitäten bei der bekannten getrennten Verwendung von Methan bzw. den höheren gas- und/oder dampfförmigen Kohlenwasserstoffen zu erwarten gewesen wäre. Bei der erfindungsgemäßen Verwendung einer Mischung von Methan und höheren gas- und/oder dampfförmigen Kohlenwasserstoffen als Einsatzstoff geht somit im Vergleich zur getrennten Verwendung der Einsatzstoffe der Verbrauch an Einsatzstoff zurück, d.h es wird eine höhere Ausbeute an Acetylen erzielt.

Als Methan wird für das erfindungsgemäße Verfahren im allgemeinen Erdgas verwendet, wie es z.B. von den Gasgesellschaften über Leitungsnetze zur Verfügung gestellt wird. Als höhere gas- und/oder dampfförmige Kohlenwasserstoffe kommen z.B. Ethan, Propan, Butan, Flüssiggas (LPG), Leichtbenzin in Betracht. Die höheren gas- und/oder dampfförmigen Kohlenwasserstoffe werden dem Methan in Mengen von 10 bis 70 Gew.%, vorzugsweise 10 bis 60 Gew.%, insbesondere 20 bis 50 Gew.%, bezogen auf die Mischung aus Methan und den höheren gas- und/oder dampfförmigen Kohlenwasserstoffen, zugemischt.

Die erhaltene Mischung aus Methan und den höheren Kohlenwasserstoffen wird anschließend auf Temperaturen von mindestens 450°C, vorzugsweise mindestens 550°C, insbesondere mindestens 570°C vorgewärmt. Die obere Grenze der Vorwärmtemperaturen für das Kohlenwasserstoffgemisch ist dadurch vorgegeben, daß das vorgewärmte Kohlenwasserstoffgemisch nach der Vermischung mit dem vorgewärmten Sauerstoff auf dem Wege von der Mischstelle mit dem Sauerstoff bis zum Zündort im Reaktionsraum des Brenners keine Vorzündung eingehen darf. In der Regel beträgt daher die Vorwärmtemperatur des Kohlenwasserstoffgemisches weniger als ca. 650°C.

Das nach der unvollständigen Verbrennung des Kohlenwasserstoffgemisches erhaltene acetylenhaltige Spaltgas wird nach Verlassen des Reaktionsraumes von Reaktionstemperaturen von 1300 bis 1700°C durch Abschrecken mit einer Abschreckflüssigkeit wie Wasser oder Kohlenwasserstofföle, z.B. aromatische Kohlenwasserstoffe enthaltende Öle, auf Temperaturen von zweckmäßig weniger als 300°C abgekühlt.

Aus dem abgekühlten acetylenhaltigen Spaltgas wird das Acetylen anschließend zweckmäßig durch Extraktion mit einem selektiven Lösungsmittel, z.B. N-Methylpyrrolidon, abgetrennt.

Die folgenden Beispiele veranschaulichen die Erfindung.

### Beispiel 1

In einer Anlage zur Herstellung von Acetylen durch unvollständige Verbrennung von Kohlenwasserstoffen mit Sauerstoff in einem Brenner wurden 6359 Nm³/h einer auf 620°C vorgewärmten Mischung aus Erdgas und Ethan, die durch Zudosieren von Ethan zum Erdgas in einer Menge von 25,5 Gew.% Ethan, bezogen auf die Mischung aus Erdgas und Ethan, erhalten worden war, mit 4083 Nm/h auf 620°C vorgewärmten Sauerstoff vermischt, und das erhaltene Kohlenwasserstoff-Sauerstoff-Gemisch wurde im Reaktionsraum des Brenners in einer Flammenreaktion umgesetzt. Die Produktion an Acetylen in dem Brenner betrug in 24 Stunden (Tagesproduktion) 30,5 t. Der spezifische Verbrauch an Sauerstoff je t produziertes Acetylen betrug 3 215 Nm³. Der spezifische Verbrauch an Kohlenwasserstoffen (Erdgas plus Ethan) betrug 4128 kg je t produziertes Acetylen.

### Vergleichsversuch 1

Wurde dagegen in der Acetylenanlage anstelle der Mischung aus Erdgas und Ethan nur Erdgas eingesetzt, betrug der spezifische Erdgasverbrauch pro t Acetylen 5 900 Nm³ (= 4 300 kg), der spezifische Sauerstoffverbrauch pro t Acetylen 3 500 Nm³ und die Kapazität des Brenners 28 t Acetylen pro Tag.

### Vergleichsversuch 2

Wurde weiter in der Acetylenanlage anstelle der Mischung aus Erdgas und Ethan nur Ethan eingesetzt, betrug der spezifische Ethanverbrauch 3 100 Nm³ (= 4 150 kg) pro t Acetylen, der spezifische Sauerstoffverbrauch 3 600 Nm³ pro t Acetylen und die Kapazität des Brenners 32 t Acetylen pro Tag.

Aufgrund der Ergebnisse der Vergleichsversuche 1 und 2 wäre rein rechnerisch für die Acetylenherstellung gemäß Beispiel 1 ein spezifischer Verbrauch an Kohlenwasserstoffen von 5170 Nm³ (= 4265 kg) je t produziertes Acetylen zu erwarten gewesen. überraschenderweise war gemäß Beispiel 1 der spezifische Kohlenwasserstoffverbrauch mit 4128 kg je t produziertes Acetylen um 3,2 % niedriger als der rechnerisch erwartete Verbrauch und der spezifische Sauerstoffverbrauch mit 3 215 Nm³ je t produziertes Acetylen um 8,8 % niedriger als der rechnerisch erwartete Verbrauch von 3225 Nm³.

### Beispiel 2

Man verfuhr wie in Beispiel 1 beschrieben, wobei jedoch eine auf 600°C vorgewärmte Mischung aus 4533 Nm³/h Erdgas und 1 500 Nm³/h Ethan (= 37,8 Gew.% Ethan, bezogen auf die Mischung aus Erdgas und Ethan) eingesetzt wurde. Die Sauerstoffmenge betrug wieder 4 083 Nm³/h. Die Tagesproduktion an Acetylen betrug 31,8 t. Der spezifische Sauerstoffverbrauch betrug 3 080 Nm³ pro t Acetylen. Der spezifische Verbrauch an Kohlenwasserstoffen (Erdgas plus Ethan) betrug 4011 kg je t produziertes Acetylen.

Aufgrund der Vergleichsversuche 1 und 2 wäre rein rechnerisch je t produziertes Acetylen ein spezifischer Verbrauch an Kohlenwasserstoffen von 4245 kg, d.h. ein um 5,8 % höherer Kohlenwasserstoffverbrauch, und ein spezifischer Sauerstoffverbrauch von 3538 Nm³, d.h. ein um 14,9 % höherer Sauerstoffverbrauch, zu erwarten gewesen.

### Beispiel 3

Man verfuhr wie in Beispiel 1 beschrieben, wobei jedoch anstelle der Erdgas/Ethan-Mischung eine auf 580°C vorgewärmte Mischung aus 5947 Nm³/h Erdgas und 1 000 kg/h Butan (= 18,7 Gew.% Butan, bezogen auf die Mischung aus Erdgas und Butan) eingesetzt wurde. Die Sauerstoffmenge betrug wieder 4 083 Nm³/h. Die Tagesproduktion an Acetylen betrug 30,7 t. Der spezifische Sauerstoffverbrauch betrug 3 192 Nm³ pro t Acetylen. Der spezifische Verbrauch an Kohlenwasserstoffen (Erdgas plus Butan) betrug 4175 kg je t Acetylen.

### Vergleichsversuch 3

Wurde in der Acetylenanlage anstelle der Mischung aus Erdgas und Butan nur Butan eingesetzt, betrug der spezifische Butan-Verbrauch 4 500 kg pro t Acetylen, der spezifische Sauerstoffverbrauch 3 400 Nm³ pro t Acetylen und die Kapazität des Brenners 32 t Acetylen pro Tag.

Aufgrund der Vergleichsversuche 1 und 3 wäre rein rechnerisch für die Acetylenherstellung gemäß Beispiel 3 je t Acetylen ein spezifischer Verbrauch an Kohlenwasserstoffen von 4342 kg, d.h. ein um 4 % höherer Kohlenwasserstoffverbrauch, und ein spezifischer Sauerstoffverbrauch von 3482 Nm³, d.h. ein um 9 % höherer Sauerstoffverbrauch, zu erwarten gewesen.

## Patentansprüche

1. Verfahren zur Herstellung von Acetylen durch unvollständige Verbrennung von Methan mit Sauerstoff, wobei das Methan und der Sauerstoff getrennt vorgewärmt und gemischt werden und die Mischung der vorgewärmten Gase in einer Flammenreaktion umgesetzt wird, dadurch gekennzeichnet, daß dem Methan höhere gas- und/oder dampfförmige Kohlenwasserstoffe in Mengen von 10 bis 70 Gew.%, bezogen auf die Mischung aus Methan und den höheren gas- und/oder dampfförmigen Kohlenwasserstoffen, zugemischt werden und die Mischung aus Methan und den höheren Kohlenwasserstoffen auf mindestens 450°C vorgewärmt wird.

## Claims

1. A process for preparing acetylene by partial combustion of methane with oxygen, wherein the methane and the oxygen are separately preheated and then mixed and the mixture of the preheated gases is reacted in a flame reaction, which comprises mixing into the methane a higher gaseous or vaporous hydrocarbon in an amount of from 10 to 70% by weight, based on the mixture of methane and the higher gaseous or vaporous hydrocarbon, and preheating the mixture of methane and a higher hydrocarbon to not less than 450°C.

## Revendications

1. Procédé de préparation d'acétylène par combustion incomplète de méthane avec de l'oxygène, le méthane et l'oxygène étant préchauffés séparément et mélangés et le mélange des gaz préchauffés étant mis en réaction à la flamme, caractérisé en ce qu'on ajoute, au méthane, des hydrocarbures supérieurs à l'état de gaz et/ou de vapeur dans des proportions de 10 à 70% en poids par rapport au mélange de méthane et des hydrocarbures supérieur: à l'état de gaz et/ou de vapeur, et en ce que le mélange de méthane et des hydrocarbures supérieurs est préchauffé à 450°C au moins.
